# EUROPEAN PATENT APPLICATION

(11) **EP 4 477 140 A1**
(43) Date of publication of application: **18.12.2024**
(21) Application number: 24182567.8
(22) Date of filing: 17.06.2024
(51) Int. Cl.: A61B 5/00, A61B 5/024, A61B 5/0205, A61B 5/01

(54) **FOOT BIOMETRIC SENSOR SYSTEM**

(30) Priority: 16.06.2023 US 202318336166
(71) Applicant: Hamilton Sundstrand Space Systems International, Inc., Windsor Locks, CT 06096 (US)
(72) Inventor: RADAWIEC, Rochelle, Vernon, CT, 06066 (US); O'CONNOR, Bradley, Rome, PA, 18837 (US); LUKER, Kelly, Glastonbury, CT, 06033 (US); SNEAD, Kelsey, Burlington, VT, 05401 (US); MCCAULEY, Devin, Leadville, CO, 80461 (US); STRICKLAND, Julie, Houston, TX, 77062 (US)
(74) Representative: Dehns

(57) **Abstract**

A biometric monitoring system for monitoring the health of user includes an article of clothing (102) configured for placement over or contacting a foot of user. The article includes: a body portion (104); a transparent sole portion (106) connected to the body portion and arranged such than the body portion is place over the foot of the user the transparent sole portion is arranged on or near a sole of the foot of the user; a sensor bed (110) arranged and configured to contact the sole portion; and one or more biometric sensors (112) on the sensor bed arranged and configured to transmit light through the transparent sole portion so that it contacts the sole of the foot of the user and receive one or more reflected signals from the sole of the foot of the user to measure the health of the user.

## Description

### BACKGROUND

Exemplary embodiments pertain to the art of space sensors and, in particular, to a system that locates a sensor or sensors at or near a foot of user of a space suit.

In some environments and applications, an atmospheric suit is used not only for protection against impacts but also to maintain a habitable environment. In a space application, including but not limited to an atmospheric suit referred to as an extravehicular mobility unit (EMU), for example, includes a helmet and full body suit supported by the primary life support system (PLSS) that are essential for maintaining an environment that sustains the astronaut.

As part of such suits, it is common to have sensors to facilitate monitoring the health and safety of the wearer. In prior systems, such data was usually collected from sensors that are affixed to the skin of the astronaut.

### BRIEF DESCRIPTION

In one embodiment, a biometric monitoring system for monitoring the health of user is disclosed. The system includes an article of clothing configured for placement over or contacting a foot of user. The article of clothing includes: a body portion; a transparent sole portion connected to the body portion and arranged such than the body portion is place over the foot of the user the transparent sole portion is arranged on or near a sole of the foot of the user; a sensor bed arranged and configured to contact the sole portion; and one or more biometric sensors on the sensor bed arranged and configured to transmit light through the transparent sole portion so that it contacts the sole of the foot of the user and receive one or more reflected signals from the sole of the foot of the user to measure the health of the user.

The biometric sensors can be optical sensors.

In embodiments, the transparent sole portion can be completely transparent or can be partially transparent and allows light from the optical sensors to pass through it.

In embodiments, the system can further include a flap arranged such that the sensor bed can be partially surrounded by and secured within the flap when it is contacting or near the transparent sole portion.

In embodiments, the biometric sensors are optical sensors.

In embodiments, the sensors can measure one or more of temperature and heart rate.

Also disclosed is method of monitoring a health of user. The method can be used with any system described above or otherwise herein it. The method can include: placing an article of clothing on a foot of user, the article including: a body portion; and a transparent sole portion connected to the body portion and arranged such than the body portion is place over the foot of the user the transparent sole portion is arranged on or near a sole of the foot of the user; providing a sensor bed that includes one or more biometric sensors; arranging the sensor bed relative to the article of clothing such that the biometric sensors transmits light through the transparent sole portion so that it contacts the sole of the foot of the user and receive one or more reflected signals from the sole of the foot of the user to measure.

In embodiments, the biometric sensors can be optical sensors.

In embodiments, the sensors can measure one or more of temperature and heart rate.

In embodiments, the transparent sole portion can be completely transparent or can be partially transparent and allows light from the optical sensors to pass through it.

In embodiments, the method can further arranging a flap on the article such that the sensor bed can be partially surround by and secured within the flap when it is contacting or near the transparent sole portion.

### BRIEF DESCRIPTION OF THE DRAWINGS

The following descriptions should not be considered limiting in any way. With reference to the accompanying drawings, like elements are numbered alike:
FIG. 1 shows portions of a system according to one embodiment that includes an article of footwear (e.g. a sock) and a sensor bed; and
FIG. 2 shows an example of sensor bed according to one embodiment;
FIGs. 3A and 3B shows different locations of a sensor bed relative to the user's foot and the article of footwear; and
FIG. 4 shows an article of footwear having a flap according to one embodiment.

### DETAILED DESCRIPTION

A detailed description of one or more embodiments of the disclosed apparatus and method are presented herein by way of exemplification and not limitation with reference to the Figures.

As previously noted, it is common for astronauts to have sensors attached to their skin while in space (either in an spacecraft or while in a spacesuit) to facilitate health monitoring. Such sensors in the space context typically require that the user shave an area of the skin and then affix the sensor to the skin.

Disclosed herein are systems and method of monitoring conditions of the user of the space suit without requiring that the user shave or that the sensors are directly attached to the skin. In one embodiment, the system is arranged to take biometric readings of the user at a location on a sole (or other portion) of one or both of the user's feet. This can be accomplished, for example, by providing a foot covering (e.g., a sock) that includes a region on the sole through which light can pass light passing region on the sole thereof. This can allow for optical sensors to sense user biometric information with the user having to shave or without the sensor having to be connected to the skin of the user. From the teachings herein it shall be understood that such a system can be useful in the context of all space suit use but, in particular, in the context of space tourism.

Herein certain sensors are discussed. These sensors can be any type of sensor that can monitor the health or other attributes of a wearer of the space suit. In a particular embodiment, the sensors are optical sensors that can measure any of heart rate, temperature, physical activity levels, blood pressure, breathing rate, oxygen levels or similar attributes. Such optical sensors can be referred to as biometric sensors from time to time herein. An optical sensor as the term is used herein are sensors that transmit light to contact the skin of a person and make determinations about the health of the person (e.g., temperature, heart rate, etc.) based on signals (e.g. light) reflected back from the skin. For instance, the sensors in the sock/insole could send an optical signal (beam) to the sole to the sole of the foot and the reflection back could be interpreted as blood pressure or oxygenation.

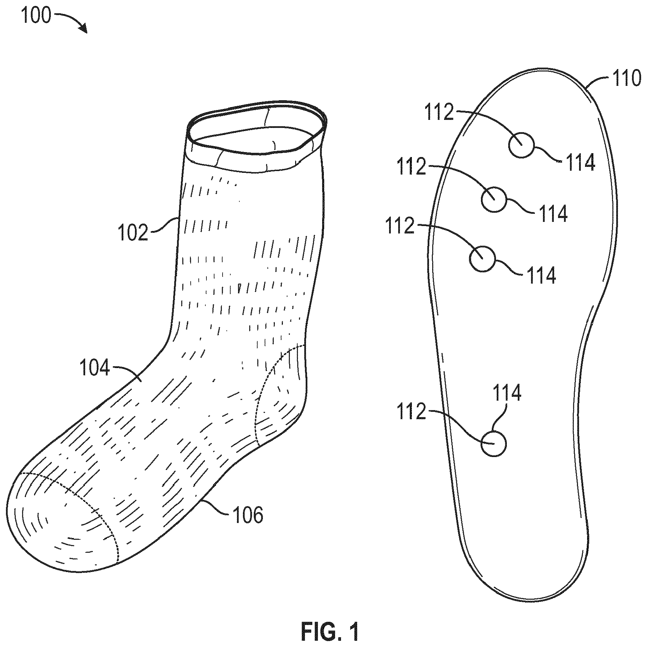

FIG. 1 shows an example system 100 according to one embodiment. The system includes an article of footwear 102. The article of footwear 102 can be implemented as a sock in one embodiment and will be referred to as such herein for brevity but other articles could be utilized. The sock 102 can be shaped an arranged so that it fits over the foot of user. The sock 102 can be formed generally of an type of suitable material subject to the discussion below.

The sock 102 includes a body portion 104 and a sole portion 106. The body portion 104 is shaped and arranged such that it can be put on a foot of the user such that it contacts or otherwise surrounds portions of the foot. As noted above, the sock 102 can be formed generally of any type of suitable material including, without limitation, cotton, wool, compression fabric, etc., and combinations thereon.

The sole portion 106 is attached to the body portion 104. The sole portion 106 is arranged such that it is adjacent to or contacts a portion of a sole of the user's foot when the sock 102 is on the user's foot.

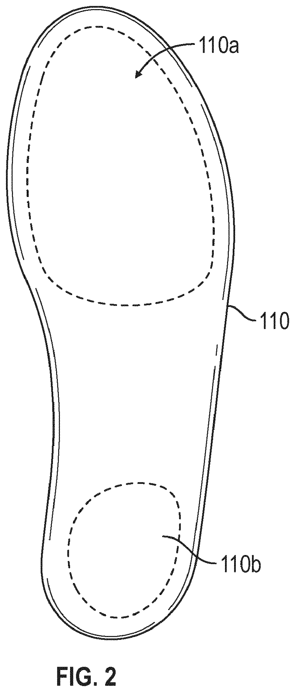

The system 100 also includes a sensor bed 110 that includes one or more biometric sensors 112. The sensor bed 110 can be an insole as shown in FIG. 1 but other shapes are contemplated. For example, the sensor bed 110 could be part of either or both portions 110a, 110b shown in FIG. 2 where the portions are separate but collectively or separately can form a sensor bed 110 and could also be in other portions of the system 100.

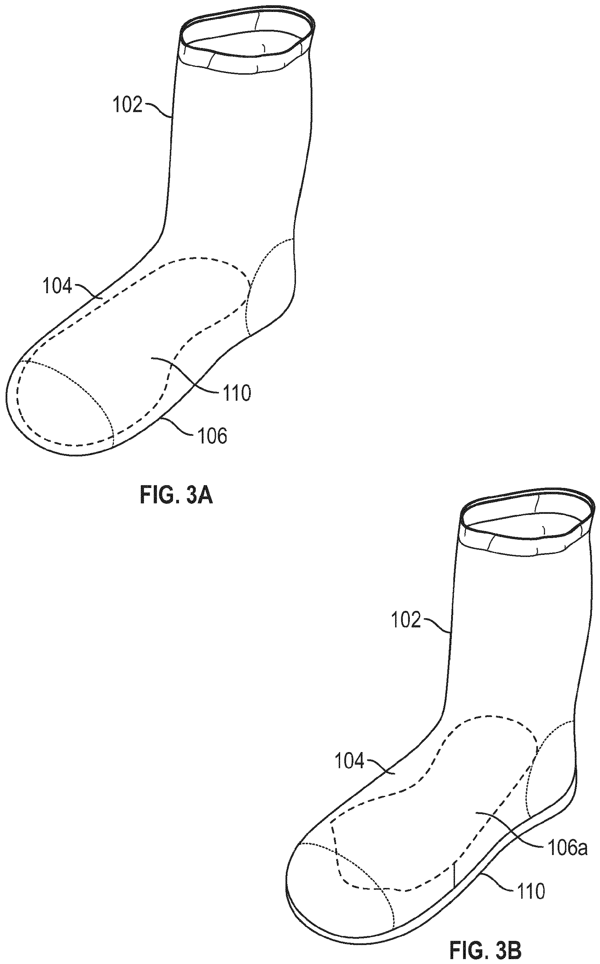

The biometric sensors 112 in the sensor bed 110 can be any type of sensor that emits one or more signals that contact the skin and receive a reflection back. The sensor bed 110 and/or the sensors 112 can be in direct contact with skin in one embodiment. In such case, the sensor bed 110 can be location between the user's foot and the sole portion 106 in one embodiment as shown in FIG. 3. That is, the sensor bed 110 is inside the sock in FIG. 3A.

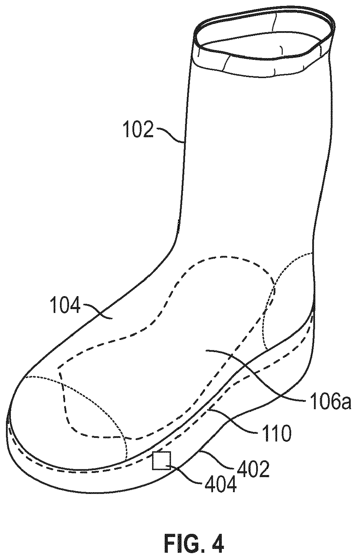

In another embodiment, the sole portion 106 is between sensor bed 110 and the user's foot as shown in FIG. 3B. In one embodiment, the sole portion 106 includes a transparent section 106a through which light (or other signals) can pass to allow the sensors to take biometric readings. The section 106a can be completely or partially transparent as long as it allows light or other signals from the sensors to gather information from the sole of the user's foot. The transparent section can be an "outer surface" of the sock 102 or it can be located such that it covered by an outer flap 402 as shown in FIG. 4. By providing the outer flap 402, the sensor bed 110 can be held in place between the flap 402 and the transparent section 106a. The flap 402 can be permanently affixed or have a portion that opens to receive the sensor bed 110. For example, the flap 402 could include one or more fasteners 404. The fasteners can allow at least a portion of the flap 402 to be removably attached/detached from other parts of the sock 102 to make inserting the sensor bed 110 easier. The fasteners 404 could be hook and loop fasteners in one embodiment. The various attachment embodiments discussed showcase a sock with a reusable insert. The insert may be used by different people at different times, such as for multiple space tourists.

In any of the above embodiments, and as shown in FIG. 1, the sensors 112 can be connected to a controller 114. The connections can either be wired as shown or wireless. The controller 114 can be configured to receive information from the sensors 114 and wirelessly transmit the information to another location. The controller 114 can interpret the information or can pass raw data or can do a combination of both.

The terminology used herein is for the purpose of describing particular embodiments only and is not intended to be limiting of the present disclosure. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, element components, and/or groups thereof.

While the present disclosure has been described with reference to an exemplary embodiment or embodiments, it will be understood by those skilled in the art that various changes may be made without departing from the scope of the invention as defined by the claims. In addition, many modifications may be made to adapt a particular situation or material to the teachings of the present disclosure without departing from the scope of the claims. Therefore, it is intended that the present disclosure not be limited to the particular embodiment disclosed as the best mode contemplated for carrying out this present disclosure, but that the present disclosure will include all embodiments falling within the scope of the claims.

## Claims

1. A biometric monitoring system for monitoring the health of user, the system comprising:
an article of clothing (102) configured for placement over or contacting a foot of user, the article of clothing including:
a body portion (104);
a transparent sole portion (106) connected to the body portion and arranged such than the body portion is place over the foot of the user the transparent sole portion is arranged on or near a sole of the foot of the user;
a sensor bed (110) arranged and configured to contact the sole portion; and
one or more biometric sensors (112) on the sensor bed arranged and configured to transmit light through the transparent sole portion so that it contacts the sole of the foot of the user and receive one or more reflected signals from the sole of the foot of the user to measure the health of the user.

2. The system of claim 1, wherein the biometric sensors are optical sensors.

3. The system of claim 2, wherein the sensors can measure one or more of temperature and heart rate.

4. The system of claim 2, wherein the transparent sole portion is completely transparent, or wherein the transparent sole portion is partially transparent and allows light from the optical sensors to pass through it.

5. The system of any preceding claim, further comprising:
a flap (402) arranged such that the sensor bed can be partially surrounded by and secured within the flap when it is contacting or near the transparent sole portion.

6. A method of monitoring a health of user, the method comprising:
placing an article of clothing (102) on a foot of user, the article including: a body portion (104); and a transparent sole portion (106) connected to the body portion and arranged such than the body portion is place over the foot of the user the transparent sole portion is arranged on or near a sole of the foot of the user;
providing a sensor bed (110) that includes one or more biometric sensors (112);
arranging the sensor bed relative to the article of clothing such that the biometric sensors transmits light through the transparent sole portion so that it contacts the sole of the foot of the user and receive one or more reflected signals from the sole of the foot of the user to measure.

7. The method of claim 6, wherein the biometric sensors are optical sensors.

8. The method of claim 6 or 7, wherein the sensors can measure one or more of temperature and heart rate.

9. The method of claim 6, 7 or 8, wherein the transparent sole portion is completely transparent.

10. The method of any of claims 6 to 9, wherein the transparent sole portion is partially transparent and allows light from the optical sensors to pass through it.

11. The method of any of claims 6 to 10, wherein the sensors can measure one or more of temperature and heart rate.

12. The method of any of claims 6 to 11, further comprising:
arranging a flap (402) on the article such that the sensor bed can be partially surround by and secured within the flap when it is contacting or near the transparent sole portion.
